(19) Europäisches Patentamt
European Patent Office
Office européen des brevets

(11) Veröffentlichungsnummer: **0 027 646**
**B1**

(12) # EUROPÄISCHE PATENTSCHRIFT

(45) Veröffentlichungstag der Patentschrift: **07.03.84**

(51) Int. Cl.³: **C 07 D 235/32**, C 07 D 235/30

(21) Anmeldenummer: **80106321.5**

(22) Anmeldetag: **17.10.80**

(54) **Verfahren zur Herstellung von Benzimidazolderivaten.**

(30) Priorität: **19.10.79 HU CI001975**
**22.04.80 HU CI001975**
**11.07.80 HU CI001975**
**19.03.80 HU CI000635**
**17.09.80 HU CI001387**
**03.06.80 HU CI001387**

(43) Veröffentlichungstag der Anmeldung: **29.04.81 Patentblatt 81/17**

(45) Bekanntmachung des Hinweises auf die Patenterteilung: **07.03.84 Patentblatt 84/10**

(84) Benannte Vertragsstaaten: **AT CH DE FR GB IT LI**

(56) Entgegenhaltungen:
**EP - A - 0 003 951**
**DE - A - 2 041 324**
**DE - A - 2 363 348**
**DE - A - 2 454 632**

**Die Akte enthält technische Angaben, die nach dem Eingang der Anmeldung eingereicht wurden und die nicht in dieser Patentschrift enthalten sind.**

(73) Patentinhaber: **CHINOIN Gyogyszer és Vegyészeti Termékek Gyára RT., To utca 1-5, H-1045 Budapest V (HU)**

(72) Erfinder: **Gönczi, Csaba, Dr. Dipl.Ing.Chem., Lisznyai u.5, H-1016 Budapest (HU)**
Erfinder: **Korbonits, Dezsö, Dr. Dipl.Ing.Chem, Verhalom u.27/D, H-1025 Budapest (HU)**
Erfinder: **Pálosi, Endre, Dipl.Ing.Chem., Pasaréti ut 114/a, H-1026 Budapest (HU)**
Erfinder: **Kiss, Pál, Dr. Dipl.Ing.Chem., Vetés u.82, H-1046 Budapest (HU)**
Erfinder: **Héja, Gergely, Dr. Dipl.Ing.Chem., Sollner u.27, H-1131 Budapest (HU)**
Erfinder: **Kun, Judit, Dipl.Ing.Chem., Gyenes u.17, H-1032 Budapest (HU)**
Erfinder: **Szomor, Mária, geb.Wundele Dipl.Ing.Chem., Fö u.21, H-1011 Budapest (HU)**
Erfinder: **Szvoboda, Ida, geb.Kanzel Dipl.Ing.Chem., Fogarasi u.85 1/5, H-1141 Budapest (HU)**
Erfinder: **Márványos, Ede, Dipl.Ing.Chem., Wesselényi u. 69, H-1077 Budapest (HU)**
Erfinder: **Horvath, Karoly, Dr. Dipl.Ing.Chem., Szakasits A. u.58/A, H-1115 Budapest (HU)**

(74) Vertreter: **Lotterhos, Hans Walter, Dr.-Ing., Lichtensteinstrasse 3, D-6000 Frankfurt am Main 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



EP 0 027 646 B1

# Verfahren zur Herstellung von Benzimidazolderivaten

Die Erfindung betrifft ein einfaches und in industriellem Massstab durchführbares Verfahren zur Herstellung von Benzimidazol-Derivaten der allgemeinen Formel I

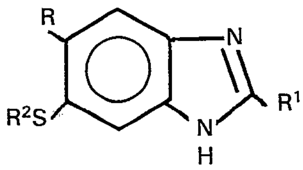

(I)

worin

R Wasserstoff, Halogen, $C_{1-4}$-Alkyl, $C_{1-3}$-Alkoxy oder Trifluormethyl.

$R^1$ Amino oder $C_{1-4}$-Alkoxycarbonylamino und

$R^2$ ein ionisch gebundenes Metallatom, ein ionisch oder kovalent gebundenes Wasserstoffatom, $C_{1-6}$-Alkyl, Alkenyl oder Alkinyl mit jeweils bis zu 6 C-Atomen oder Cyclohexyl, Benzyl oder 2-Phenyläthyl bedeuten, sowie deren Salzen.

Benzimidazol-Derivate des beanspruchten Typs sind bekannt. Sie stellen wertvolle Anthelmintica dar, die in der Human- und in der Veterinärmedizin verwendet werden. Eine besonders grosse Bedeutung kommt dabei dem [5(6)-Propylthio]-2-benzimidazolyl-carbaminsäuremethylester (Albendazol) zu.

Die Herstellung dieser Verbindungsgruppe ist z.B. in den US-PSs 3 480 642, 3 574 485, 3 915 986, 3 956 499, 4 080 461, 4 152 522, der DE-PS 2 364 351, den DE-OSs 2 041 324, 2 363 348, 2 454 632 und in der EP-Anmeldung 0 003 951 beschrieben.

Allen diesen Verfahren ist gemeinsam, dass der Cyclisierung zum Benzimidazolmolekül der allgemeinen Formel I ein 1,2-Diamino-benzol unterworfen wird, das in 4-Stellung bereits die fertig ausgebildete Alkylthio-gruppe enthält. Die Unterschiede zwischen diesen Verfahren des Standes der Technik bestehen nur in der Synthese der in 4-Stellung durch die Alkylthiogruppe substituierten 1,2-Diamino-benzole.

Nach den Verfahren der US-PSs 3 915 986, 3 956 499, DE-OSs 2 041 324 und 2 363 348 wird die Alkylthiogruppe durch Umsetzung der toxischen und übelriechenden Alkylmercaptane mit dem Chloratom des 2-Acetamido- bzw. 2-Amino-4-chlor-nitrobenzols in die 4-Stellung des 2-Acetamido- bzw. 2-Amino-nitrobenzols eingeführt und das so erhaltene 2-Acetamido- bzw. 2-Amino-4-alkylthio-nitrobenzol durch Hydrolyse und Reduktion in das 1,2-Diamino-4-alkylthio-benzol übergeführt. Der Nachteil dieser Verfahrensführung besteht darin, dass nicht nur die Umsetzung mit Alkylmercaptan in sehr mässiger Ausbeute verläuft (etwa 40%, US-PS 3 956 499), sondern auch die Herstellung des als Ausgangsmaterial verwendeten 2-Acetamido-4-chlor-nitrobenzols. Das 2-Acetamido-4-chlor-nitrobenzol wird durch Nitrierung von 2-Chlor-acetanilid erhalten, wobei sich als Nebenprodukt das isomere 2-Chlor-4-nitro-acetanilid bildet, das nicht nur die Ausbeute vermindert, sondern auch abgetrennt werden muss [J. Org. Chem. 12, 799 (1947) und 42, 554 (1977)].

Nach den Verfahren der US-PS 4 080 461 und der DE-OSs 2 363 351, 2 454 632; Ber. 59, 190 (1926); J. Chem. Soc. 1928, 1364, wird zur Herstellung des 1,2-Diamino-4-alkylthio-benzols in sehr umständlicher Verfahrensweise zunächst das aus Anilin durch Rhodanierung erhaltene 4-Thiocyano-anilin nach Acetylierung durch Nitrierung in 2-Nitro-4-thiocyano-acetanilid übergeführt, aus dem durch selektive Reduktion und Alkylierung 2-Nitro-4-alkylthio-acetanilid und dann durch Hydrolyse und Reduktion 1,2-Diamino-4-alkylthio-benzol erhalten wird.

Zur Umgehung dieser mehrstufigen Verfahrensweise ist in der US-PS 4 152 522 und der EP-Anmeldung 0 003 951 vorgeschlagen worden, von dem durch Rhodanierung des o-Nitro-anilins erhaltenen 4-Thiocyano-2-nitro-anilin auszugehen, das mit einem Alkalicyanid und einem Alkylhalogenid zum 4-Alkylthio-2-nitroanilin umgesetzt und durch Reduktion in das 1,2-Diamino-4-thiocyano-benzol übergeführt wird.

Die Umwandlung der Thiocyangruppe in die Alkylthiogruppe ist aber nur in speziellen Fällen möglich und erfordert besondere Bedingungen, da sich sehr leicht Nebenprodukte, hauptsächlich Disulfide, bilden. Sie kann nicht in Gegenwart von Gruppen, die zur Hydrolyse neigen, angewandt werden. Nach dem Verfahren der DE-PS 2 363 351 wird z.B. das 1-Acetamino-2-nitro-4-thiocyano-benzol in Methanol bei einer Temperatur von 18 bis 20° C mit Kaliumhydroxyd und Propylbromid umgesetzt. In diesem Fall ist aber die Bildung der Propylthiogruppe von der Hydrolyse der Acetylgruppe begleitet, so dass die Aminogruppe erneut acyliert werden muss. Nach dem Verfahren der US-PS 4 152 522 und der EP-Anmeldung 0 003 951 werden die schonenderen Alkali- oder Erdalkalimetallcyanide anstelle von Kaliumhydroxyd zur Durchführung dieser Reaktionen verwendet, wobei die Alkali- bzw. Erdalkalimetallcyanide und das Propylhalogenid in sehr hohem Überschuss eingesetzt werden, der in die Mutterlaugen geht und zur Bildung von Dicyan als Nebenprodukt führt.

Das wesentliche Merkmal dieser bekannten Verfahren besteht darin, die Ausbildung der Alkylthiogruppe in dem entsprechenden Benzolderivat vor der Cyclisierung zu dem gegenüber verschiedenen chemischen Einwirkungen empfindlichen Benzimidazolyl-alkylcerbamat durchzuführen.

Nach dem Verfahren der DE-OS 2 820 375 wird die Alkylthiogruppe in der letzten Verfahrensstufe durch Reduktion der entsprechenden bis-Benzimidalzol-disulfide mit nachfolgender Alkylierung ausgebildet.

Obgleich die bekannten Verfahren zur Herstellung der Verbindungen vom Albendazol-typ sehr umständlich waren und nur mässige Ausbeuten lieferten, ist, trotz der Bedeutung, die diese Verbindungsgruppe wegen ihrer anthelmintischen Wirkung hat, kein Versuch unternommen worden, die Thiocyangruppe eines [5(6)-Thiocyano]-2-benzimidazolyl--carbamats in die Alkylthiogruppe überzuführen. Dies ist überraschend, weil die [5(6)-Thiocyano]-2--benzimidazolyl-carbamate bereits aus der DE-PS 2 363 351 und der US-PS 4 080 461 bekannt waren. Der Grund dafür dürfte sein, dass man wegen der bekannten Empfindlichkeit sowohl der Thiocyangruppe als auch der Carbamatgruppe bei Eingriffen am [5(6)-Thiocyano]-2-benzimidazolyl-carbamat--molekül Zersetzungen an diesen beiden empfindlichen Gruppen befürchtete.

Es wurde überraschender Weise gefunden, dass die Benzimidazol-Derivate der allgemeinen Formel I

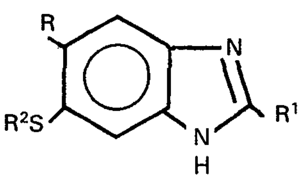 (I)

worin

R, $R^1$ und $R^2$ die oben angegebene Bedeutung haben, sowie deren Salze, in einfacher Weise, ausgezeichneten Ausbeuten und sehr reinem Zustand hergestellt werden können, wenn man eine Verbindung der allgemeinen Formel II

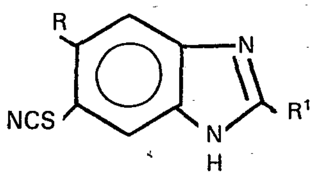 (II)

worin

R und $R^1$ die oben angegebene Bedeutung haben, durch Behandlung mit einer nucleophilen organischen oder anorganischen Base in die Verbindung der allgemeinen Formel III

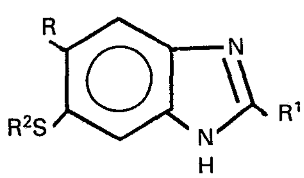 (III)

worin

R und $R^1$ die oben angegebene Bedeutung haben und

$R^{2'}$ ein ionisch gebundenes Metallatom — vorzugsweise ein Alkali- oder Erdalkalimetallatom — oder ein ionisch oder kovalent gebundenes Wasserstoffatom bedeutet, überführt, die gegebenenfalls mit einem Alkylierungsmittel der allgemeinen Formel IV

$$R^{2''}-Q \quad (IV),$$

worin

$R^{2''}$ $C_{1-6}$-Alkyl, Alkenyl oder Alkinyl mit jeweils bis zu 6 C-Atomen, Cyclohexyl, Benzyl oder 2-Phenyläthyl und

Q Hydroxy, Halogen, 1/2 $SO_4$, 1/3 $PO_3$, 1/3 $PO_4$, $SO_3C_6H_5$ oder $SO_3C_6H_4CH_3$ bedeuten, in an sich bekannter Weise umgesetzt wird, und gewünschtenfalls die so erhaltene Verbindung der allgemeinen Formel I in ihre Salze überführt.

Als nucleophile organische oder anorganische Basen können z.B. Alkalimetall- oder Erdalkalimetallhydroxyde, -carbonate, -alkoxyde oder -aryloxyde, Ammoniumhydroxyd, tertiäre Amine, Alkalimetall- oder Erdalkalimetallazide, -cyanide, vorzugsweise Alkalimetall- oder Erdalkalimetallsulfide oder -hydrogensulfide, insbesondere Natriumsulfid, verwendet werden.

Als Alkylierungsmittel werden $C_{1-3}$-Alkohole, Alkylhalogenide, vorteilhaft Alkylbromide, Alkylsulfate, Alkylphosphite, Alkylphosphate, Benzolsulfonsäure- oder p-Toluolsulfonsäure-alkylester, wobei der Alkylrest jeweils 1-6 C-Atome umfasst, bevorzugt.

Die Reaktion wird zweckmässig in Gegenwart von Wasser und/oder einem organischen Lösungsmittel durchgeführt. Als organisches Lösungsmittel können mit Wasser mischbare organische Lösungsmittel, vorzugsweise $C_{1-3}$-Alkohole (z.B. Methanol, Äthanol, Isopropanol, n-Propanol), Aceton, Dioxan, Dimethylformamid, Pyridin, Dimethylsulfoxyd oder deren Gemische verwendet werden.

Die Reaktion kann auch in Wasser und in mit Wasser nicht-mischbaren organischen Lösungsmitteln, vorzugsweise chlorierten Kohlenwasserstoffen, insbesondere Tetrachlorkohlenstoff oder Chlorbenzole, oder Kohlenwasserstoffen, insbesondere Toluol oder Aryläther, wie Anisol oder Diphenyläther, und in Gegenwart eines Phasentransferkatalysators durchgeführt werden. Als Phasentransferkatalysator können Tetrabutylammoniumchlorid, Trimethylbezylammoniumchlorid und Triäthylbenzylammoniumchlorid eingesetzt werden.

Die Umsetzung hängt von den Eigenschaften der Reaktanten ab und wird zweckmässig bei einer Temperatur zwischen 0 und 100°C bzw. dem Siedepunkt des Reaktionsgemisches, vorzugsweise zwischen 10 und 40°C, durchgeführt. Werden als Alkylierungsmittel Alkohole verwendet, liegt die Reaktionstemperatur zweckmässig zwischen 80 und 100°C.

Nach einer vorteilhaften Ausführungsform des Verfahrens wird die Umsetzung der Verbindungen der allgemeinen Formel II mit dem Alkylierungsmittel und der nucleophilen Base gleichzeitig in einem Reaktionsgefäss durchgeführt.

Man kann aber auch so verfahren, dass man die Verbindungen der allgemeinen Formel II zunächst mit dem Alkylierungsmittel der allgemeinen Formel IV vermischt und dann erst die nucleophile Base zufügt.

Zweckmässig werden die Verbindungen der allgemeinen Formel II und die nucleophilen Base in äquimolaren Mengen eingesetzt, da unter den Bedingungen praktisch keine Nebenreaktionen auftreten und eine nahezu quantitative Ausbeute erzielt wird. Dies ist darauf zurückzuführen, dass beim Arbeiten mit stöchiometrischen Mengen der störende Effekt der Sulfid- bzw. der gebildeten Rhodanid-Ionen praktisch nicht zur Geltung kommt. Es bilden sich keine Disulfide und andere Nebenprodukte, und die anwesende Alkylcarbamatgruppe erleidet keine Zersetzung.

Die als Ausgangsverbindungen verwendeten Verbindungen der allgemeinen Formel II

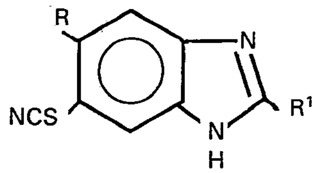

(II)

worin

R und $R^2$ die oben angegebene Bedeutung haben, sind leicht in hoher Ausbeute und sehr reinem Zustand zugänglich:

a) durch die selektive Rhodanierung, d.h. durch Umsetzung von Benzimidazol-Derivaten der allgemeinen Formel

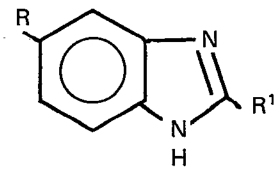

worin

R und $R^1$ die oben angegebene Bedeutung haben, mit Chlorgas und einem Rhodanid der allgemeinen Formel

$R^3$—SCN,

worin

$R^3$ ein Alkali- oder Erdalkalimetallatom oder die Ammoniumgruppe bedeutet, und

b) durch Reaktion von Verbindungen der allgemeinen Formel

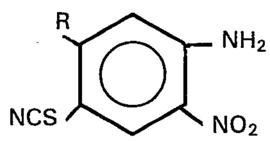

mit Eisen zur entsprechenden 1,2-Diaminoverbindung, die entweder mit einem gegebenenfalls durch $C_{1-4}$-Alkoxycarbonyl substituierten Cyanamid oder einem Halogencyan zu dem Benzimidazol-Derivat der allgemeinen Formel II cyclisiert werden.

Beide Verfahren können auch in industriellem Massstab durchgeführt werden.

Das Verfahren der Erfindung lässt sich — auch wegen der leichten Zugänglichkeit der Ausgangsverbindungen der allgemeinen Formel II — in industriellem Massstab durchführen. Ausserdem liefert es keine Umweltschutzprobleme.

Die Verbindungen der allgemeinen Formel I können mit organischen oder anorganischen Säuren in die entsprechenden Säureadditionssalze übergeführt werden.

Weitere Einzelheiten des Verfahrens gemäss Erfindung sind den nachstehenden Beispielen zu entnehmen.

*Beispiel 1*

2,48 g 5(6)-Thiocyano-2-(methoxycarbonyl-amino)-benzimidazol werden in 25 ml 96%igem Äthanol suspendiert und 1,25 g Propylbromid zugefügt. Danach tropft man unter kräftigem Rühren innerhalb einer halben Stunde bei einer Temperatur von 20 bis 25°C 2,4 g Natriumsulfid-nonahydrat in 2 ml Wasser zu. Es bildet sich eine gelbe Lösung, aus der nach kurzer Zeit die Kristallausscheidung beginnt. Das Produkt wird abfiltriert, gewaschen und getrocknet.

Es werden 2,50 g (94% Ausbeute) 5(6)-Propylthio-2-(methoxycarbonyl-amino)-benzimidazol erhalten; Fp.: 212 bis 213°C.

*Beispiel 2*

124 g 5(6)-Thiocyano-2-(methoxycarbonyl-amino)-benzimidazol suspendiert man in 1,5 Liter 96%igem Äthanol, gibt 62,5 g Propylbromid zu und tropft dann unter kräftigem Rühren innerhalb einer halben Stunde bei einer Temperatur von 20 bis 25°C eine Lösung von 120 g Natriumsulfid-nonahydrat in 200 ml Wasser zu. Es bildet sich eine braune Lösung, aus der nach kurzer Zeit die Kristallausscheidung beginnt. Das Produkt wird abfiltriert, zuerst mit Wasser, danach mit Methanol gewaschen und getrocknet. Es werden 127 g (96% Ausbeute) 5(6)-Propylthio-2-(methoxycarbonyl-amino)-benzimidazol erhalten. Fp.: 212 bis 213°C.

*Beispiel 3*

24,8 g 5(6)-Thiocyano-2-(methoxycarbonyl-amino)-benzimidazol suspendiert man in 100 ml Dimethylsulfoxyd, tropft unter Rühren innerhalb von 3 bis 5 Minuten bei einer Temperatur von 20°C eine Lösung von 29,0 g Natriumsulfid-nonahydrat in 100 ml Methanol zu und versetzt die erhaltene reine mattgelbe Lösung bei der gleichen Temperatur mit 15 g Propylbromid. Nach 1 bis 2 Minuten beginnt eine Kristallausscheidung. Das Gemisch wird noch eine halbe Stunde weiter gerührt, wonach der pH-Wert des Gemisches mit 5 n Salzsäure auf 7 eingestellt wird. Die Kristalle werden abfiltriert, zuerst mit Wasser und danach mit Alkohol gewaschen und im Vakuum bei einer Temperatur von 60°C getrocknet.

Es werden 26,0 g (98% Ausbeute) 5(6)-Propylthio-2-(methoxycarbonyl-amino)-benzimidazol erhalten. Fp.: 214 bis 215°C.

*Beispiele 4 bis 15*

In der in den Beispielen 1 bis 3 beschriebenen Weise werden unter Einsatz der entsprechenden Ausgangsstoffe folgende Verbindungen der allgemeinen Formel I hergestellt:

($R^5$ = Methoxycarbonyl-amino)

| Beispiel | $R^1$ | $R^6$ | Fp. (°C) |
|---|---|---|---|
| 4 | Chlor | n-Propylthio | 250 (unter Zers.) |
| 5 | Chlor | Benzylthio | 234 bis 236 |
| 6 | Chlor | Allylthio | 203 bis 205 |
| 7 | Chlor | Propinylthio | 305 bis 307 |
| 8 | Chlor | Äthylthio | 237 bis 238 |
| 9 | Chlor | Cyclohexyl | 294 bis 295 |
| 10 | Brom | n-Propylthio | 191 bis 193 |
| 11 | Methyl | n-Propylthio | 234 bis 235 (unter Zers.) |
| 12 | Methoxy | n-Propylthio | 296 bis 298 |
| 13 | Butyl | n-Propylthio | 202 bis 204 |
| 14 | Trifluormethyl | n-Propylthio | 252 |
| 15 | Fluor | n-Propylthio | 252 bis 253 |

*Beispiel 16*

Einer Lösung von 2,48 g 5(6)-Thiocyano-2-(methoxycarbonyl-amino)-benzimidazol in 60 ml 85%igem Aceton wird eine Lösung von 2,9 g Natriumsulfid-nonahydrat in 5 ml Wasser zugesetzt, nach 40 Minuten das erhaltene Reaktionsgemisch entfärbt und danach filtriert. Zu der erhaltenen gelben Lösung wird Aceton gegeben, worauf sich das Natriumsalz des 5(6)-Thiol-2-(methoxycarbonyl-amino)-benzimidazols abscheidet. Das Produkt wird abfiltriert, mit Aceton gewaschen und im Vakuum über Kaliumhydroxyd getrocknet.
Es werden 2,3 g erhalten; Fp.: 300 bis 305°C (unter Zersetzung).

*Beispiel 17*

2,3 g des nach Beispiel 16 hergestellten Natriumsalzes werden in 25 ml 50%igem Alkohol aufgenommen. Der pH-Wert des Gemisches wird mit Salzsäure auf 5 eingestellt und das ausgefallene 5(6)-Thiol-2-(methoxycarbonyl-amino)-benzimidazol abfiltriert und gewaschen.

So erhält man 1,65 g des Produktes, das bei 261 bis 262°C schmilzt.
Analyse:

| | | |
|---|---|---|
| berechnet: | N = 18,82% | S = 14,36% |
| gefunden: | N = 18,30% | S = 14,35%. |

IR.: 1.705 $cm^{-1}$ (Ester-carbonyl)
3.150 bis 2.200 $cm^{-1}$ (Benzimidazol-N)
802 $cm^{-1}$ (1,2,4-trisubstituiertes Benzol)
H-NMR: ein abwechselbares Proton bei 6,388 ppm.
Raman: charakteristische Bande für die SH-Gruppe bei 2.555 $cm^{-1}$.

*Beispiel 18*

2,45 g Natriumsalz des 5(6)-Thiol-2-(methoxycarbonyl-amino)-benzimidazols werden in 35 ml 65 Vol.%igem Aceton gelöst. Die entstandene Lösung wird entfärbt und filtriert. Danach setzt man bei einer Temperatur von 10 bis 15°C 1,3 g Propylbromid zu, stellt nach einer halben Stunde den pH-Wert des Gemisches auf 6 ein, filtriert dann das ausgefallene Produkt ab und wäscht es. Es werden 2,5 g 5(6)-Propylthio-2-(methoxycarbonyl-amino)-benzimidazol erhalten; Fp.: 214°C (unter Zersetzung).

*Beispiel 19*

2,23 g 5(6)-Thiol-2-(methoxycarbonyl-amino)-benzimidazol werden in 30 ml, 0,8 g Natriumhydroxyd enthaltendem, 60 Vol.%igem Alkohol gelöst, und danach bei einer Temperatur von 10 bis 15°C mit 1,3 g Propylbromid versetzt. Nach halbstündigem Rühren wird der pH-Wert des Gemisches auf 6 eingestellt und das ausgeschiedene 5(6)-Propylthio-2-(methoxycarbonyl-amino)-benzimidazol abfiltriert.
Ausbeute: 2,4 g; Fp.: 212 bis 214°C (unter Zersetzung).

*Beispiel 20*

49,66 g (0,2 Mol) 5(6)-Thiocyano-2-(methoxycarbonyl-amino)-benzimidazol suspendiert man in 600 ml 65 Vol.%igem wässrigem Aceton und fügt der erhaltenen Suspension innerhalb von 5 Minuten bei einer Temperatur von 20 bis 25°C eine Lösung von 58 g (0,24 Mol) Natriumsulfid-nonahydrat in 100 ml Aceton zu, wobei eine hellgelbe Lösung mit wenig Niederschlag entsteht. Diese Lösung wird mit 4 g Aktivkohle entfärbt, filtriert und zweimal mit je 20 ml 65 Vol.%igem Aceton gewaschen. Der erhaltenen Lösung wird innerhalb von 5 Minuten bei einer Temperatur von 10 bis 15°C eine Lösung von 30 g (0,24 Mol) Propylbromid in 100 ml Aceton zugefügt, das erhaltene Gemisch eine Stunde gerührt und danach der pH-Wert mit 5 n Salzsäure auf 6 eingestellt. Nach halbstündigem Rühren wird das ausgefallene Produkt abfiltriert, zweimal in je 60 ml 50 Vol.%igem Aceton suspendiert und danach zweimal mit je 30 ml Aceton gewaschen und getrocknet.

Es werden 47,3 g (Ausbeute 89%) 5(6)-Propylthio-2-(methoxycarbonyl-amino)-benzimidazol in Form von schneeweissen Mikrokristallen erhalten.

*Beispiel 21*

24,8 g (0,1 Mol) 5(6)-Thiocyano-2-(methoxycarbonyl-amino)-benzimidazol suspendiert man unter

kräftigem Rühren in 300 ml 65 Vol.%igem, wässrigem Aceton, gibt 30 g Propylbromid zu und gibt der erhaltenen Suspension bei einer Temperatur von 15°C 22 g Kaliumhydroxyd in Form einer 40%igen wässrigen Lösung zu, wobei nach 1 bis 2 Minuten eine hellgelbe Lösung entsteht. Das Endprodukt der Reaktion wird durch dünnschichtchromatographische Analyse bestimmt. (Platte: Silicagel $G_{254}$; Lösungsmittelgemisch: Chloroform - Essigsäure 9 : 1; Entwickler: UV-Strahl). Nach Beendigung der Reaktion (3 bis 4 Stunden) wird das Gemisch entfärbt, filtriert und danach der pH-Wert der Lösung mit 5 n Salzsäure auf 6 eingestellt. Nach kurzem Stehen werden die ausgefallenen Kristalle abfiltriert, zunächst mit wässrigem Aceton und dann mit Wasser gewaschen.

Es werden 20,5 g 5(6)-Propylthio-2-(methoxycarbonyl-amino)-benzimidazol erhalten; Fp.: 210 bis 212°C.

*Beispiel 22*

Es wird in der in Beispiel 21 angegebenen Weise verfahren, jedoch mit dem Unterschied, dass anstelle von 65%igem Aceton, 70%iges wässriges Äthanol verwendet wird.

So werden 19,8 g 5(6)-Propylthio-2-(methoxycarbonyl-amino)-benzimidazol erhalten; Fp.: 209 bis 211°C.

*Beispiel 23*

Es wird wie in Beispiel 21 gearbeitet, jedoch mit dem Unterschied, dass anstelle von Kaliumhydroxyd, 32 g Natriumhydroxyd in Form einer 40%igen wässrigen Lösung verwendet werden.

So erhält man 19,7 g 5(6)-Propylthio-2-(methoxycarbonyl-amino)-benzimidazol; Fp.: 211 bis 213°C.

**Patentansprüche**

1. Verfahren zur Herstellung von Benzimidazol-Derivaten der allgemeinen Formel I

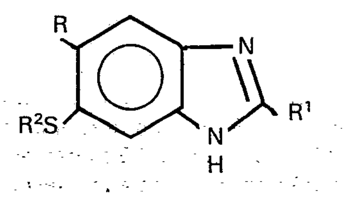
(I)

worin

R Wasserstoff, Halogen, $C_{1-4}$-Alkyl, $C_{1-3}$-Alkoxy oder Trifluormethyl,

$R^1$ Amino oder $C_{1-4}$-Alkoxycarbonylamino und

$R^2$ ein ionisch gebundenes Metallatom, ein ionisch oder kovalent gebundenes Wasserstoffatom, $C_{1-6}$-Alkyl, Alkenyl oder Alkinyl mit jeweils bis zu 6 C-Atomen oder Cyclohexyl, Benzyl oder 2-Phenyläthyl bedeuten, sowie deren Salzen, dadurch gekennzeichnet, dass man eine Verbindung der allgemeinen Formel II

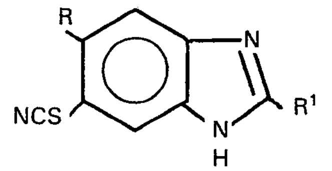
(II)

worin

R und $R^1$ die oben angegebene Bedeutung haben, durch Behandlung mit einer nucleophilen organischen oder anorganischen Base in die Verbindung der allgemeinen Formel III

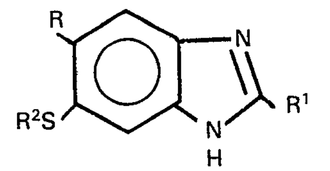
(III)

worin

R und $R^1$ die oben angegebene Bedeutung haben und

$R^{2'}$ ein ionisch gebundenes Metallatom oder ein ionisch oder kovalent gebundenes Wasserstoffatom bedeutet, überführt, die gegebenenfalls mit einem Alkylierungsmittel der allgemeinen Formel IV

$$R^{2''}-Q \qquad (IV)$$

worin

$R^{2''}$ $C_{1-6}$-Alkyl, Alkenyl oder Alkinyl mit jeweils bis zu 6 C-Atomen, Cyclohexyl, Benzyl oder 2-Phenyläthyl und

Q Hydroxy, Halogen, 1/2 $SO_4$, 1/3 $PO_3$, 1/3 $PO_4$, $SO_3C_6H_5$ oder $SO_3C_6H_4CH_3$ bedeuten, in an sich bekannter Weise umgesetzt wird und gewünschtenfalls die so erhaltene Verbindung der allgemeinen Formel I in ihre Salze überführt.

2. Verfahren nach Anspruch 1, dadurch gekennzeichnet, dass in der verwendeten Verbindung der allgemeinen Formel III das ionisch gebundene Metallatom ein Alkalimetall- oder Erdalkalimetallatom bedeutet.

3. Verfahren nach Anspruch 1 oder 2, dadurch gekennzeichnet, dass man die Behandlung mit einer nucleophilen Base und einem Alkylierungsmittel nacheinander oder gleichzeitig durchführt.

4. Verfahren nach Anspruch 1 bis 3, dadurch gekennzeichnet, dass man als nucleophile organische oder anorganische Basen, Alkalimetall- oder Erdalkalimetallhydroxyde, -carbonate, -alkoxyde, -aryloxyde, -azide, -cyanide, -sulfide oder -hydrogensulfide, Ammoniumhydroxyd oder Amine verwendet.

5. Verfahren nach Anspruch 4, dadurch gekennzeichnet, dass man als nucleophile anorganische Base Natriumsulfid oder Kaliumhydroxyd verwendet.

6. Verfahren nach Anspruch 1 bis 5, dadurch gekennzeichnet, dass man als Alkylierungsmittel der allgemeinen Formel IV $C_{1-3}$-Alkohole, Alkylhalogenide, Alkylsulfate, Alkylphosphite, Alkylphosphate, Benzolsulfonsäure- oder p-Toluolsulfonsäurealkylester, wobei der Alkylrest jeweils 1-6 C-Atome umfasst, verwendet.

7. Verfahren nach Anspruch 1 bis 6, dadurch gekennzeichnet, dass man die Reaktion in Wasser

und/oder in einem organischen Lösungsmittel durchführt.

8. Verfahren nach Anspruch 7, dadurch gekennzeichnet, dass man als organische Lösungsmittel $C_{1-3}$-Alkohole, Aceton, Dioxan, Dimethylformamid, Dimethylsulfoxyd, Pyridin, oder deren Gemisch, verwendet.

9. Verfahren nach Anspruch 1 bis 6, dadurch gekennzeichnet, dass man die Reaktion in einem mit Wasser nicht-mischbaren organischen Lösungsmittel in Gegenwart eines Phasentransferkatalysators durchführt.

10. Verfahren nach Anspruch 9, dadurch gekennzeichnet, dass man als mit Wasser nicht-mischbare organische Lösungsmittel Kohlenwasserstoffe, chlorierte Kohlenwasserstoffe oder Aryläther verwendet.

**Claims**

1. Process for preparing benzimidazole derivatives of general formula I

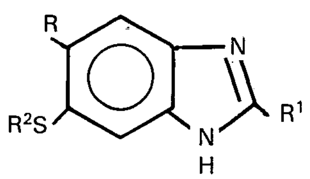

(I)

wherein

R represents hydrogen, halogen, $C_{1-4}$ alkyl, $C_{1-3}$ alkoxy or trifluormethyl,

$R^1$ represents amino or $C_{1-4}$ alkoxycarbonylamino and

$R^2$ represents an ionically bonded metal atom, an ionically or covalently bonded hydrogen atom, $C_{1-6}$ alkyl, alkenyl or alkynyl each having up to 6 carbon atoms, or cyclohexyl, benzyl or 2-phenylethyl, and the salts thereof, characterised in that a compound of general formula II

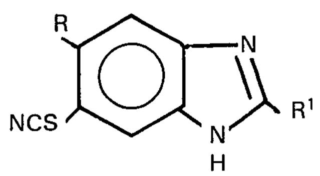

(II)

wherein

R and $R^1$ are as hereinbefore defined, is converted, by treatment with a nucleophilic organic or inorganic base, into a compound of general formula III

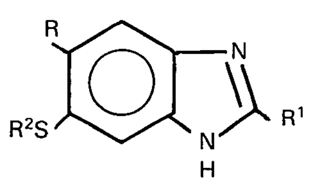

(III)

wherein

R and $R^1$ are as hereinbefore defined and

$R^{2'}$ represents an ionically bonded metal atom or an ionically or covalently bonded hydrogen atom, which is optionally reacted with an alkylating agent of general formula IV

$$R^{2''}-Q \qquad (IV)$$

wherein

$R^{2''}$ represents $C_{1-6}$ alkyl, alkenyl or alkynyl each having up to 6 carbon atoms, cyclohexyl, benzyl or 2-phenylethyl and

Q represents hydroxy, halogen, 1/2 $SO_4$, 1/3 $PO_3$, 1/3 $PO_4$, $SO_3C_6H_5$ or $SO_3C_6H_4CH_3$, in a manner known per se, and if desired the compound of general formula I thus obtained is converted into a salt thereof.

2. Process as claimed in claim 1, characterised in that, in the compound of general formula III used, the ionically bonded metal atom is an alkali metal or alkaline earth metal atom.

3. Process as claimed in claim 1 or 2, characterised in that the treatment with a nucleophilic base and with an alkylating agent is carried out successively or simultaneously.

4. Process as claimed in claim 1 to 3, characterised in that the nucleophilic organic or inorganic bases used are alkali metal or alkaline earth metal hydroxides, carbonates, alkoxides, aryloxides, azides, cyanides, sulphides or hydrogen sulphides, ammonium hydroxide or amines.

5. Process as claimed in claim 4, characterised in that sodium sulphide or potassium hydroxide is used as the nucleophilic inorganic base.

6. Process as claimed in claims 1 to 5, characterised in that $C_{1-3}$ alcohols, alkyl halides, alkyl sulphates, alkyl phosphites, alkyl phosphates, alkyl benzenesulphonates or alkyl p-toluenesulphonates, wherein the alkyl group contains 1 to 6 carbon atoms, are used as the alkylating agent of general formula IV.

7. Process as claimed in claims 1 to 6, characterised in that the reaction is carried out in water and/or in an organic solvent.

8. Process as claimed in claim 7, characterised in that $C_{1-3}$ alcohols, acetone, dioxan, dimethylformamide, dimethylsulphoxide, pyridine or a mixture thereof is used as the organic solvent.

9. Process as claimed in claims 1 to 6, characterised in that the reaction is carried out in a water-immiscible organic solvent in the presence of a phase transfer catalyst.

10. Process as claimed in claim 9, characterised in that hydrocarbons, chlorinated hydrocarbons or aryl ethers are used as the water-immiscible organic solvent.

**Revendications**

1. Procédé de préparation de dérivés du benzimidazole de formule générale I:

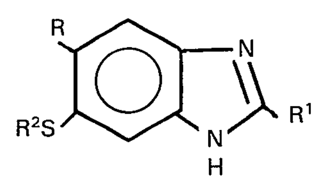

(I)

dans laquelle:

R représente l'hydrogène, un halogène, un groupe alkyle en $C_1$-$C_4$, alcoxy en $C_1$-$C_3$ ou trifluorométhyle,

$R^1$ représente un groupe amino ou (alcoxy en $C_1$-$C_4$)-carbonylamino, et

$R^2$ représente un atome de métal à liaison ionique, un atome d'hydrogène à liaison ionique ou covalente, un groupe alkyle en $C_1$-$C_6$, alcényle ou alcynyle contenant chacun jusqu'à 6 atomes de carbone ou cyclohexyle, benzyle ou 2-phényléthyle, et de leurs sels, caractérisé en ce que l'on convertit un composé de formule générale II:

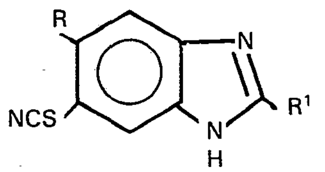

(II)

dans laquelle R et $R^1$ ont les significations indiquées ci-dessus, par traitement à l'aide d'une base organique ou minérale nucléophile, en le composé de formule générale III:

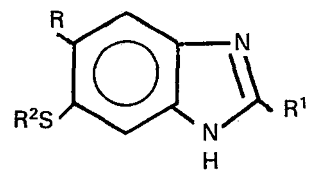

(III)

dans laquelle:

R et $R^1$ ont les significations indiquées ci-dessus, et

$R^{2'}$ représente un atome de métal à liaison ionique ou un atome d'hydrogène à liaison ionique ou covalente, qu'on fait réagir éventuellement, de manière connue en soi, avec un agent alkylant de formule générale IV:

$$R^{2''}—Q \qquad (IV)$$

dans laquelle:

$R^{2''}$ représente un groupe alkyle en $C_1$-$C_6$, alcényle ou alcynyle contenant chacun jusqu'à 6 atomes de carbone, cyclohexyle, benzyle ou 2-phényléthyle, et

Q représente un groupe hydroxy, un halogène, 1/2 $SO_4$, 1/3 $PO_3$, 1/3 $PO_4$, $SO_3C_6H_5$ ou $SO_3C_6H_4CH_3$, et, si on le désire, on convertit le composé obtenu, répondant à la formule générale I, en ses sels.

2. Procédé selon la revendication 1, caractérisé en ce que, dans le composé de formule générale III utilisé, l'atome de métal à liaison ionique est un atome de métal alcalin ou alcalino-terreux.

3. Procédé selon la revendication 1 ou 2, caractérisé en ce que le traitement par une base nucléophile et le traitement par un agent alkylant sont effectués successivement ou simultanément.

4. Procédé selon l'une quelconque des revendications 1 à 3, caractérisé en ce que l'on utilise en tant que bases organiques ou minérales nucléophiles des hydroxydes, carbonates, alcoolates, phénates, azothydrates, cyanures, sulfures ou bisulfures de métaux alcalins ou alcalino-terreux, l'hydroxyde d'ammonium ou des amines.

5. Procédé selon la revendication 4, caractérisé en ce que l'on utilise en tant que base minérale nucléophile le sulfure de sodium ou l'hydroxyde de potassium.

6. Procédé selon l'une quelconque des revendications 1 à 5, caractérisé en ce que l'on utilise en tant qu'agents alkylants de formule générale IV des alcools en $C_1$-$C_3$, des halogénures d'alkyle, des sulfates d'alkyle, des phosphites d'alkyle, des phosphates d'alkyle, des benzène-sulfonates ou p-toluènesulfonates d'alkyle, chacun des groupes alkyles contenant 1 à 6 atomes de carbone.

7. Procédé selon l'une quelconque des revendications 1 à 6, caractérisé en ce que la réaction est effectuée dans l'eau et/ou dans un solvant organique.

8. Procédé selon la revendication 7, caractérisé en ce que l'on utilise en tant que solvants organiques des alcools en $C_1$-$C_3$, l'acétone, le dioxane, le diméthylformamide, le diméthylsulfoxyde, la pyridine ou leurs mélanges.

9. Procédé selon l'une quelconque des revendications 1 à 6, caractérisé en ce que la réaction est effectuée dans un solvant organique non miscible à l'eau en présence d'un catalyseur à transfert de phase.

10. Procédé selon la revendication 9, caractérisé en ce que l'on utilise en tant que solvants organiques non miscibles à l'eau des hydrocarbures, des hydrocarbures chlorés ou des éthers aryliques.